# EUROPEAN PATENT SPECIFICATION

(11) **EP 1 582 154 B1**
(45) Date of publication and mention of the grant of the patent: **26.11.2008**
(21) Application number: 05251943.6
(22) Date of filing: 29.03.2005
(51) Int. Cl.: A61B 17/12

(54) **Vascular occlusive device with elastomeric bioresorbable coating**
Gefässverschliessende Spirale mit elastomerischer Schicht
Spirale d'occlusion vasculaire comprenant une couche élastomérique

(30) Priority: 29.03.2004 US 811753
(43) Date of publication of application: 05.10.2005
(73) Proprietor: Cordis Neurovascular, Inc., Miami Lakes, Florida 33014 (US)
(72) Inventor: Jones, Donald K., Lauderhill Florida 33351 (US); Roller, Mark B., North Brunswick New Jersey 08902 (US); Scopelianos, Angelo G., Whitehouse Station New Jersey 08889 (US); Vyakarnam, Murty N., South Orange New Jersey 07079 (US)
(74) Representative: Tunstall, Christopher Stephen

(56) References cited:
- US-A- 5 468 253
- US-A1- 2001 034 531
- US-A1- 2003 004 568

## Description

### BACKGROUND OF INVENTION

### Field of the Invention

The present invention relates to medical implantable device, and more particularly, to a vascular occlusive device, such as an embolic coil for occluding an aneurysm, which includes an elastomeric, bioresorbable coating placed on the occlusive device for reacting with bodily tissue in order to promote a desired result, for example, promoting controlled tissue ingrowth into the occlusive device and into the aneurysm.

### Description of the Prior Art

For many years vasculature occlusive devices have been placed within the vasculature of the human body to occlude, or partially occlude, blood flow through the vasculature. Additionally, such devices have been introduced into aneurysms in order to fill, or partially fill, the aneurysm so as to reduce the pressure which is applied to the interior of the aneurysm in order to prevent further growth or expansion of the aneurysm. These devices may take the form of a coil, such as a helical coil, and are typically placed within the vessel or aneurysm by use of a delivery catheter which is inserted into the vessel and positioned such that the distal end of the delivery catheter is adjacent to a selected site for placement. Once the occlusive device is placed within a blood vessel or aneurysm, surrounding tissue reacts with the ''foreign" object and begins to grow into and around the device to provide more complete occlusion of the aneurysm. Examples of such delivery catheters are disclosed in U.S. Patent No. 5,108,407, entitled "Method And Apparatus For Placement Of An Embolic Coil" and U.S. Patent No. 5,122,136, entitled "Endovascular Electrolytically Detachable Guidewire Tip For The Electroformation Of Thrombus In Arteries, Veins, Aneurysms, Vascular Malformations And Arteriovenous Fistulas." These patents disclose catheter systems for delivering embolic coils to preselected positions within vessels of the human body in order to treat aneurysms, or alternatively, to occlude a blood vessel at a preselected location.

Occlusive devices which take the form of coils may be helically wound coils, random wound coils, coils wound within coils or other such coil configurations. Examples of various coil configurations are disclosed in U.S. Patent No. 5,334,210, entitled, "Vascular Occlusion Assembly" and U.S. Patent No. 5,382,259, entitled, "Vasoocclusion Coil With Attached Tubular Woven Or Braided Fibrous Covering." Such coils are generally formed from radiopaque metallic materials, such as platinum, gold, tungsten or alloys of these metals. Oftentimes several coils are placed at a given location within a vessel, or within an aneurysm, to more completely occlude, or partially occlude, the flow of blood through the vessel or aneurysm. Thrombus growth onto the coils further enhances the occlusive effect of the coils.

In the past, embolic coils have been placed within the distal end of a delivery catheter and when the distal end of the catheter is properly positioned, the coil may then be pushed out of the end of the catheter with, for example a guidewire, to release the coil at the desired location. This procedure of placement of the embolic coil is conducted under fluoroscopic visualization such that the movement of the coil may be monitored and the coil may be placed at a desired location.

In addition, such coils have been specifically designed to be stretch resistant, such as the vasculature occlusive coil disclosed in U.S. Patent No. 5.853,418, entitled, "Stretch Resistant Vaso-Occlusive Coils (II)" which discloses a helically wound coil having a polymeric stretch resistant member extending through the lumen of the coil and fixedly attached to both ends of the coil to prevent the coil from stretching.

In order to increase the thrombogenicity of an embolic coil, such coils have included a coating, such as collagen, which is applied to the surface of the coil. This concept is disclosed in U.S. Patent No. 5,690,671, entitled, "Embolic Elements And Methods And Apparatus For Their Delivery," which discloses such a collagen coated embolic coil. One of the problems with collagen coated coils is that when the coil is initially positioned within an aneurysm, there is an immediate reaction between the collagen and surrounding blood which causes an immediate thromblytic response, which can in turn make it difficult, if not impossible to reposition the coil to a more desirable location within the aneurysm.

In addition, U.S. Patent No. 5,980,550, entitled, "Water-Soluble Coating For Bioactive Vasoocclusive Devices," discloses an embolic coil having an inner coating which serves as a thrombogenic agent and an outer coating of a water soluble agent which dissolves after placement of the coil in order expose the thrombogenic inner coating to enhance the growth of thrombus into an around the coil. The water soluble coating prevents the thrombogenic inner coating from coming into contact with the surrounding blood until the water soluble coating is dissolved by contact with blood which is comprised largely of water. White the vascular occlusive device disclosed in this patent includes an agent for enhancing thromboginicity of the device and also includes an outer coating to prevent such activity until the outer coating is dissolved by blood flow, there is no control over when the dissolving process begins and therefore no control over the time in which the thrombogenic agent becomes activated. Without such control, it is possible that thrombus can begin forming on the coil prior to the time the coil is properly placed within a vessel, or aneurysm, therefore making it very difficult if not impossible to reposition, or remove, the improperly placed coil. Alternatively, with water soluble outer protective coating the passive process of removing the outer coating may be so slow that the reaction may not occur in a timely manner.

Also, sutures have been fabricated from elastomeric materials which after implantation are bioabsorbed into the body over a period of time, metabolized or eliminated from the body with no harm. Exemplary bioabsorbable, biocompatible elastomeric materials include but are not limited to elastomeric copolymers of ε-caprolactone and glycolide (including polyglycolic acid) with a mole ratio of ε-caprolactone to glycolide of from about 35:65 to about 65:35, more preferably from 45:55 to 35:65; elastomeric copolymers of ε-caprolactone and lactide (including L-lactide, D-lactide, blends thereof, and lactic acid polymers and copolymers) where the mole ratio of ε-caprolactone to lactide is from about 35:65 to about 65:35 and more preferably from 45:55 to 30:70 or from about 95:5 to about 85:15; elastomeric copolymers of p-dioxanone (1,4-dioxan-2-one) and lactide (including L-lactide, D-lactide, blends thereof, and lactic acid polymers and copolymers) where the mole ratio of p-dioxanone to lactide is from about 40:60 to about 60:40; elastomeric copolymers of ε-caprolactone and p-dioxanone where the mole ratio of ε-caprolactone to p-dioxanone is from about from 30:70 to about 70:30; elastomeric copolymers of p-dioxanone and trimethylene carbonate where the mole ratio of p-dioxanone to trimethylene carbonate is from about 30:70 to about 70:30; elastomeric copolymers of trimethylene carbonate and glycolide (including polyglycolic acid) where the mole ratio of trimethylene carbonate to glycolide is from about 30:70 to about 70:30; elastomeric copolymers of trimethylene carbonate and lactide (including L-lactide, D-lactide, blends thereof, and lactic acid polymers and copolymers) where the mole ratio of trimethylene carbonate to lactide is from about 30:70 to about 70:30); and blends thereof. Examples of suitable bioabsorbable elastomers are described in U.S. Pat. Nos. 4,045, 418; 4,057,537 and 5,468,253. One such elastomeric material is an elastomer comprised of a random copolymer of epsilon-caprolactone (ε-caprolactone) and glycolide. This specific elastomeric material is disclosed in the '253 patent.

Still further United States Patent Application Serial No. 10/738,477, filed on December 17, 2003, entitled, "Activatable Bioactive Impantable Medical Device And Method Of Use," (Attorney Docket No. CRD5046) published as US-2005-0137568 A and U.S. Patent Application Serial No. 10/738,473, filed on December 17, 2003, entitled, "Activatable Bioactive Vascular Occlusive Device And Method Of Use," (Attorney Docket No. CRD5061) published as US-2005-0149107, disclose implantable medical devices having bioactive coating with an outer barrier to prevent exposure of the bioactive coating until such time as the outer barrier is dissolved or removed by the application of an external agent.

### SUMMARY OF THE INTENTION

The present invention provides an embolic occlusion device (10) comprised of an embolic support member (12) having an elastomeric coating (20) disposed thereon, wherein the coating consists essentially of a random copolymer of: a) a first monomer selected from the group consisting of ε-caprolactone, trimethylene carbonate, an ether lactone and combinations thereof, and b) the balance of the copolymer being substantially a second monomer selected from the group consisting of lactide, glycolide, para-dioxanone and combinations thereof, and wherein the random copolymers are prepared by reacting the monomers with an initiator selected from a mono- or polyhydric alcohol, a hydroxy acid, a polyalkylene glycol and a poly-hydroxy alkane. The coating is preferably comprised essentially of a random copolymer of: a) from about 35 to about 45 weight percent of a first monomer selected from the group consisting of ε-caprolactone, trimethylene carbonate, an ether lactone and combinations thereof, and b) the balance of the copolymer being substantially a second monomer selected from the group consisting of glycolide, para-dioxanone and combinations thereof, wherein the random copolymer exhibits an inherent viscosity of from about 0.6 dL/g to about 4.0 dL/g.

The random copolymer may preferably be a copolymer of from about 40 to about 45 weight percent of ε-caprolactone, and the balance being glycolide.

The random copolymer preferably exhibits a percent crystallinity of less than about 25 percent, the random copolymer preferably exhibits an inherent viscosity of from about 1.0 g/dL to about 2.0 g/dL, the random copolymer preferably exhibits a percent elongation greater than about 200, or the random polymer preferably exhibits a percent elongation greater than about 500.

In a preferred aspect of the invention there is provided an embolic occlusion device which includes a support member, such as an embolic coil, having an elastomeric bioabsorbable coating disposed on the support member wherein the coating is comprised of a copolymer of ε-caprolactone and glycolide. Preferably, the random copolymer is a copolymer of from about 40 to about 45 weight percent of ε-caprolactone and the balance being glycolide.

Preferably, the elastomeric coating takes the form of a bioresorbable coating which bioabsorbes within the human body.

### BRIEF DESCRIPTION OF THE DRAWINGS

Figure 1 is an elevational view of an embolic coil illustrating a vascular occlusive coil in accordance with one embodiment of the present invention;
Figure 2 is an elevational view, partly in cross-section of the vascular occlusive coil as shown in Figure 1 illustrating an elastomeric, bioresorbable coating in accordance with one embodiment of the present invention;
Figures 3A through 3C illustrate the method steps of applying multiple vascular occlusive coils as shown in Figure 1 and 2 into an aneurysm and thereafter the controlled growth of fibrotic tissue within the aneurysm.

### DESCRIPTION OF THE PREFERRED EMBODIMENT

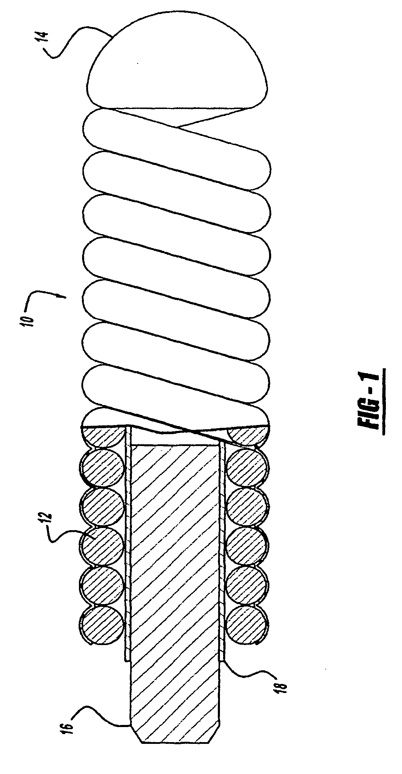

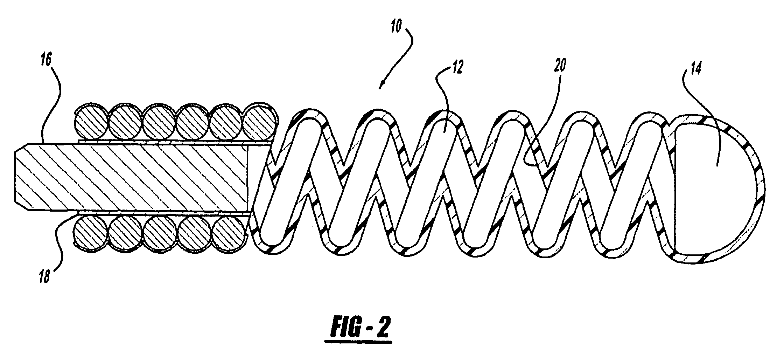

Figures 1 and 2 illustrate a vascular occlusive device which takes the form of an embolic coil 10 which may be placed along with other similar coils into a blood vessel or into an aneurysm in order to partially fill the aneurysm. More particularly, the embolic coil 10 is a typical embolic coil which comprises a helically wound coil 12 formed from a platinum alloy wire wound into a helical configuration. The diameter of the wire is generally in the range of about 0.00175 cm (0.0007 inches) to about 0.02 cm (0.008 inches) and the outer diameter of the coil 10 is preferably in a range of about 0.0075 cm (0.003 inches) to about 0.1375 cm (0.055 inches). While the particular embolic coil 10 illustrated in Figures 1 and 2 is shown as being a straight, helically wound coil, it should be appreciated that embolic coils are formed in various configurations and may take the form of a helix, a random shaped configuration or even a coil within a coil.

Preferably the embolic coil 10 includes a weld bead 14 which is attached to the distal end of the coil for providing a less traumatic distal end for the embolic coil 10. In addition, the embolic coil 10 includes a cylindrical headpiece 16 which is placed into the lumen of the helically wound coil 12 at the proximal end of the coil and is held in place by an adhesive material 18 interposed between the cylindrical headpiece 16 and the helical coil 12. The construction of the embolic coil 10 and an associated hydraulic deployment system for placing the embolic coil within an aneurysm is disclosed in more detail in U.S. Patent Application Serial No. 10/102,154, entitled. "Small Diameter Embolic Coil Hydraulic Deployment System." filed March 19, 2002, assigned to the same assignee of the present invention, published as US-2002-0151915 A.

Figure 2 illustrates in more detail an elastomeric, bioresorbable coating 20 which is disposed upon the coil 10 for modulating the tissue response in order to present a severe thrombolytic response while still inducing a mild or moderate inflammatory response to surrounding tissue, including blood, to promote controlled growth of fibrotic tissue within the aneurysm.

The coating 20, which exhibits the highly desired elastomeric properties can be prepared in accordance with the descriptions provided in U.S. Patent Nos. 5,133,739 and 4,605,730, and with the examples set forth in U.S. Patent No. 5,468,253. With respect to the teachings in these patents, each patent describes the preparation of a random copolymer of ε-caprolactone and glycolide, as an intermediate in the preparation of a crystalline segmented copolymer to be used for specific medical applications. It is the processing of this intermediate random copolymer which has led to the surprising discovery that the intermediate copolymer itself has the combination of outstanding properties, including its elastomeric properties, which make it well-suited for numerous medical applications.

The random copolymers are prepared by reacting the monomers with an initiator such as a mono- or polyhydric alcohol, e.g. diethylene glycol, trimethylol propane, or pentaerythritol; or a hydroxy acid such as lactic or glycolic acid. Other initiators which can be used include polyalkylene glycols such as triethylene glycol, and poly-hydroxy alkanes such as glycerol, mannitol, glucose and the like.

The inherent viscosity of the random copolymer is desirably greater than about 0.6, preferably within a range of from about 1.0 to about 2.0, as measured in a 0.1 gram per deciliter (g/dL) solution of the polymer in hexafluoroisopropanol (HFIP) at 25° C. If the inherent viscosity is less than about 0.6 dl/g, then the strength properties of the copolymer would most likely be inadequate for numerous medical device applications. If the inherent viscosity were greater than about 4.0 dl/g, then one may encounter significant processing difficulties in the fabrication of medical devices or components for such devices from the copolymers. This may require solution casting techniques to prepare useful products. In addition, the percent crystallinity of the random copolymer, as measured by x-ray diffraction, is advantageously less than about 25 percent, preferably less than about 15 percent. If the crystallinity of the copolymer were greater than about 25 percent, then the copolymer would be relatively stiff and non-etastomeric.

The preferred random copolymer is a copolymer of ε-caprolactone or trimethlyene carbonate, and glycolide. The most preferred random copolymer is a copolymer of ε-caprolactone and glycolide. The amount of ε-caprolactone (or trimethylene carbonate, ether lactone, or a mixture of any of these with or without ε-caprolactone, if such equivalent components are used) from which the random copolymer is composed is critical to achieve acceptable elastomeric properties in combination with good mechanical properties. The term "ether lactone" is meant to include 1,4-dioxepan-2-one, 1,5-dioxepan-2-one, substituted equivalents of these compounds, as well as the dimers of these compounds. The preferred amount of ε-caprolactone is between about 30 to about 50 weight percent. If less than 30 weight percent of ε-caprolactone is used, then the copolymer would not exhibit elastomeric properties. Additionally, it may be difficult to process such a copolymer by conventional techniques because such a copolymer may not be soluble in solvents traditionally used in medical applications. If the amount of ε-caprolactone in the random copolymer were greater than about 50 weight percent, then the strength properties of the copolymer would diminish appreciably, thus rendering the copolymer unsuitable for many applications where strength is needed, and the elastomeric properties would diminish as well. Preferably, the range of ε-caprolactone in the comonomer mixture from which the random copolymer is prepared ranges from about 30 to about 45 weight percent. Ideally, the range is from about 35 to about 45 weight percent.

Minor amounts of additives or comonomers can be added to the comonomer mixture from which the random copolymer is prepared, so long as these additional additives or comonomers do not significantly impact upon the elastomeric properties of the copolymers, or its rate of bioabsorption. For example, it may be desired to add certain components to modify or enhance the properties of the copolymer for specific applications. So long as the amount of ε-caprolactone in the comonomer mixture lies within the range from about 30 to about 50 weight percent, and the properties of the copolymer are not substantially effected, then such additional components may be used. Of course, the other primary component of the comonomer mixture in addition to ε-caprolactone is glycolide, para-dioxanone, lactide, or a mixture of these. Therefore, the term "substantially" which appears in the appended claims refers to allowing the incorporation of such minor components in addition to the balance of the copolymer composition being glycolide, para-dioxanone, lactide, or a mixture of these comonomers.

The tensile properties of an ε-caprolactone/glycolide (PCL/PGA) 45/55 by weight copolymer which is initiated with trimethylol propane or pentaerythritol are enhanced considerably when compared to diethylene glycol initiated copolymer of the same composition.

The elastomeric coating 20 may be applied to the surface of the embolic coil 10 by various conventional techniques, such as dip coating or spraying. Prior to coating, a solution is prepared by dissolving 0.1 to 20 parts of the ε-caprolactone/glycolide copolymer with a solvent, such as 1,4 dioxane. Preferably a solution could be prepared by dissolving 0.5 to 5 of the ε-caprolactone/glycolide copolymer parts with 95 to 99.5 solvent, and most preferably dissolving 1 part of the ε-caprolactone/glycolide copolymer with 99 parts solvent, such as 1,4 dioxane. The mixture is placed into a flask and stirred with a magnetic stir bar. The mixture is then gently heated to 60 ± 5 degrees centigrade and continuously stirred for a minimum of 4 hours, but not to exceed 8 hours. A clear homogenous solution is then obtained by filtering the solution through an extra coarse porosity filter (Pyrex brand extraction thimble with fritted disc) using dry nitrogen to enhance the filtration of this viscous solution. The coil 10 is then dip coated into this mixture, or alternatively the mixture is sprayed onto the coil 10.

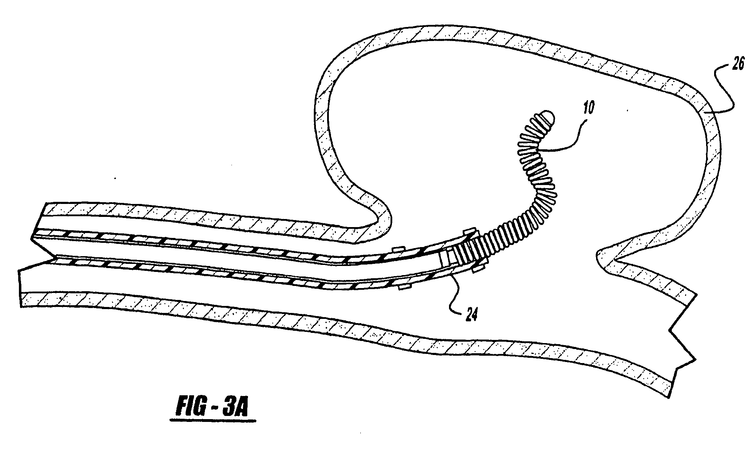

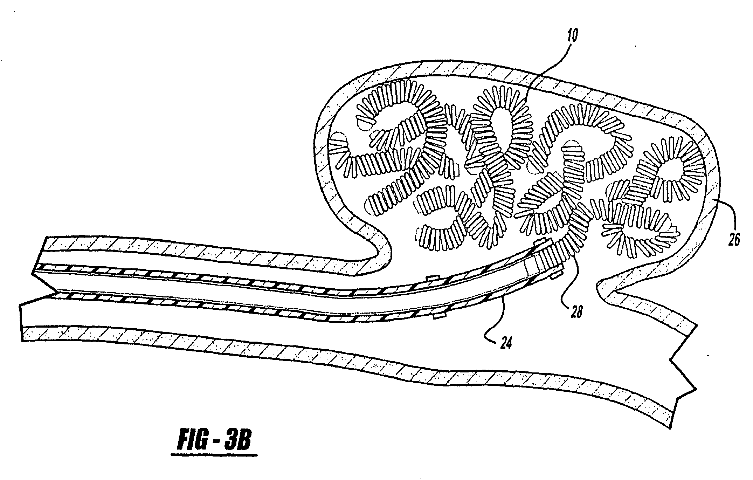

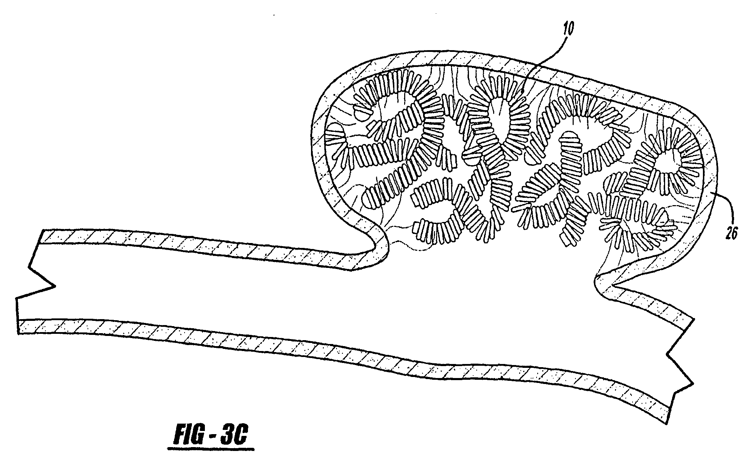

Figures 3A through 3C generally illustrate a method of utilizing the coated embolic coil 10 of the present invention. More particularly, Figure 3A illustrates a delivery catheter 24 having a coil 10 placed in the distal end of the catheter for delivery into an aneurysm 26. Figure 3B illustrates the delivery catheter 24 being used to position multiple vascular occlusive coils including a final embolic coil 28 into the aneurysm 26. Figure 3C illustrates the coils with the coating exposed to bodily tissue to thereby cause a reaction between the coil and the bodily tissue, such as blood, to in turn, cause tissue ingrowth into the coil 10 and into the aneurysm 26. As the coating 20 bioabsorbes into the body this reaction continues to thereby cause a continuation of tissue ingrowth into the coil and into the aneurysm.

As may be appreciated, the advantage of the subject invention over prior embolic devices is that there is a modulated response between the elastomeric bioabsorbable coating on the embolic device and bodily tissue, or fluids, to thereby cause controlled tissue ingrowth into the embolic device and into the aneurysm. In addition, the coating biodegrades and is ultimately absorbed into the body.

Although a preferred embodiment of the present invention has been described, it is to be understood that various modifications may be made by those skilled in the art without departing from the scope of the claims, which follow:

## Claims

1. An embolic occlusion device (10) comprised of an embolic support member (12) having an elastomeric coating (20) disposed thereon, wherein the coating consists essentially of a random copolymer of; a) a first monomer selected from the group consisting of ε-caprolactone, trimethylene carbonate, an ether lactone and combinations thereof, and b) the balance of the copolymer being substantially a second monomer selected from the group consisting of lactide, glycolide, para-dioxanone and combinations thereof, and wherein the random copolymers are prepared by reacting the monomers with an initiator selected from a mono- or polyhydric alcohol, a hydroxy acid, a polyalkylene glycol and a poly-hydroxy alkane.

2. An embolic occlusion device as defined in claim 1 wherein the coating is bioresorbable or bioabsorbable.

3. An embolic occlusion device as defined in claim 1 or 2, wherein said embolic support member is an embolic coil.

4. An embolic occlusion device as defined in claim 3, wherein said embolic coil takes the form of a helically wound coil.

5. An embolic occlusion device as defined in claim 1, wherein the first monomer is present in an amount of from 35 to 45 weight percent.

6. An embolic occlusion device as defined in any preceding claim, wherein said elastomeric bioresorbable coating consists of a random copolymer of ε-caprolactone and glycolide.

7. An embolic occlusion device as defined in claim 6, wherein the random copolymer is comprised of a copolymer of from about 35 weight percent of ε-caprolactone, and the balance being glycolide.

8. An embolic occlusion device as claimed in claim 1, wherein said elastomeric coating is comprised of a copolymer of caprolactone.

9. An embolic occlusion device as defined in claim 1, wherein the elastomeric coating is comprised of a copolymer of ε-caprolactone.

10. An embolic occlusion device as defined in claim 7, wherein the random copolymer exhibits a percent crystallinity of less than about 25 percent.

11. An embolic occlusion device as defined in claim 10, wherein the random copolymer exhibits a percent elongation greater than about 200.

12. An embolic occlusion device as defined in claim 11, wherein the random copolymer exhibits a percent elongation greater than about 500.

13. A medical device comprised of the embolic device (10) according to any one of claims 1 to 12.

## Patentansprüche

1. Eine Embolieverschlussvorrichtung (10), umfassend ein Embolieträgerelement (12) mit einer darauf angeordneten elastomeren Beschichtung (20), wobei die Beschichtung im Wesentlichen aus einem beliebigen Co-Polymer besteht, aus: a) einem ersten Monomer, gewählt aus der Gruppe, bestehend aus a) ε-Caprolacton, Trimethylenkarbonat, einem Etherlacton und Kombinationen daraus, und b) wobei der Rest zum Co-Polymer im Wesentlichen ein zweites Monomer ist, gewählt aus der Gruppe, bestehend aus Lactid, Glycolid, Para-dioxanon und Kombinationen daraus, und wobei die beliebigen Co-Polymere durch Reagieren der Monomere mit einem Initiator, gewählt aus einem eine oder mehrere Hydroxylgruppen enthaltenden Alkohol, einer Hydroxysäure, einem Polyalkylenglykol und einem Polyhydroxyalkan.

2. Embolieverschlussvorrichtung nach Anspruch 1, wobei die Beschichtung bioresorbierbar oder bioabsorbierbar ist.

3. Embolieverschlussvorrichtung nach Anspruch 1 oder 2, wobei das Embolieträgerelement eine Emboliespirale ist.

4. Embolieverschlussvorrichtung nach Anspruch 3, wobei die Emboliespirale die Form einer spiralförmig gewundenen Spirale annimmt.

5. Embolieverschlussvorrichtung nach Anspruch 1, wobei das erste Monomer in einem Umfang von 35 bis 45 Gewichtsprozent vorliegt.

6. Embolieverschlussvorrichtung nach einem der vorhergehenden Ansprüche, wobei die elastomere bioresorbierbare Beschichtung aus einem beliebigen Co-Polymer aus ε-Caprolacton und Glykolid besteht.

7. Embolieverschlussvorrichtung nach Anspruch 6, wobei das beliebige Co-Polymer ein Co-Polymer von ungefähr 35 Gewichtsprozent ε-Caprolacton umfasst und der Rest Glykolid ist.

8. Embolieverschlussvorrichtung nach Anspruch 1, wobei die elastomere Beschichtung ein Co-Polymer aus Caprolacton umfasst.

9. Embolieverschlussvorrichtung nach Anspruch 1, wobei die elastomere Beschichtung ein Co-Polymer aus ε-Caprolacton umfasst.

10. Embolieverschlussvorrichtung nach Anspruch 7, wobei das beliebige Co-Polymer eine Kristallinität von weniger als ungefähr 25% aufweist.

11. Embolieverschlussvorrichtung nach Anspruch 10, wobei das beliebige Co-Polymer eine Streckung von mehr als ungefähr 200% aufweist.

12. Embolieverschlussvorrichtung nach Anspruch 11, wobei das beliebige Co-Polymer eine Streckung von mehr als ungefähr 500% aufweist.

13. Medizinische Vorrichtung, umfassend eine Embolievorrichtung (10) nach irgendeinem der Ansprüche 1 bis 12.

## Revendications

1. Dispositif d'occlusion embolique (10) composé d'un élément de support embolique (12) ayant un revêtement élastomère (20) disposé sur celui-ci, dans lequel le revêtement est essentiellement constitué d'un copolymère aléatoire de : a) un premier monomère choisi dans le groupe comprenant ε-caprolactone, carbonate de triméthylène, un lactone d'éther et des combinaisons de ceux-ci, et b) le reste du copolymère étant sensiblement un second monomère choisi dans le groupe comprenant lactide, glycolide, para-dioxanone et combinaisons de ceux-ci, et dans lequel les copolymères aléatoires sont préparés en faisant réagir les monomères avec un initiateur choisi parmi un alcool mono ou polyhydrique, un acide hydroxylé, un polyalkylène glycol et un alcane poly-hydroxylé.

2. Dispositif d'occlusion embolique selon la revendication 1, dans lequel le revêtement est biorésorbable ou bioabsorbable.

3. Dispositif d'occlusion embolique selon la revendication 1 ou 2, dans lequel ledit élément de support embolique est une spire embolique.

4. Dispositif d'occlusion embolique selon la revendication 3, dans lequel ladite spire embolique prend la forme d'une bobine enroulée en spirale.

5. Dispositif d'occlusion embolique selon la revendication 1, dans lequel le premier monomère est présent dans une quantité de 35 à 45 % en poids.

6. Dispositif d'occlusion embolique selon l'une quelconque des revendications précédentes, dans lequel ledit revêtement biorésorbable élastomère comprend un copolymère aléatoire de ε-caprolactone et glycolide.

7. Dispositif d'occlusion embolique selon la revendication 6, dans lequel le copolymère aléatoire est composé d'environ 35 % en poids de ε-caprolactone, le reste étant du glycolide.

8. Dispositif d'occlusion embolique selon la revendication 1, dans lequel ledit revêtement élastomère est composé d'un copolymère de caprolactone.

9. Dispositif d'occlusion embolique selon la revendication 1, dans lequel le revêtement élastomère est composé d'un copolymère de ε-caprolactone.

10. Dispositif d'occlusion embolique selon la revendication 7, dans lequel le copolymère aléatoire présente une cristallinité en pourcentage inférieure à environ 25 %.

11. Dispositif d'occlusion embolique selon la revendication 10, dans lequel le copolymère aléatoire présente un allongement en pourcentage supérieur à environ 200.

12. Dispositif d'occlusion embolique selon la revendication 11, dans lequel le copolymère aléatoire présente un allongement en pourcentage supérieur à environ 500.

13. Dispositif médical composé du dispositif embolique (10) selon l'une quelconque des revendications 1 à 12.
